# EUROPÄISCHE PATENTSCHRIFT

(11) **EP 0 565 512 B1**
(45) Veröffentlichungstag und Bekanntmachung des Hinweises auf die Patenterteilung: **16.12.1998**
(21) Anmeldenummer: 93890047.9
(22) Anmeldetag: 19.03.1993
(51) Int. Cl.: C12N 9/76, C12N 9/74, C12N 11/00

(54) **Verfahren zur Aktivierung von Blutgerinnungsfaktoren**
Method of activating blood coagulation factors
Procédé pour l'activation de facteurs coagulants sanguins

(30) Priorität: 06.04.1992 AT 713/92
(43) Veröffentlichungstag der Anmeldung: 13.10.1993
(73) Patentinhaber: IMMUNO Aktiengesellschaft, A-1221 Wien (AT)
(72) Erfinder: Linnau, Yendra, Dr., A-1224 Wien (AT)
(74) Vertreter: Weinzinger, Arnulf, Dipl.-Ing.

(56) Entgegenhaltungen:
- EP-A- 0 120 835
- EP-A- 0 294 851
- MEDLINE DATABASE. U.S. NATIONAL LIBRARY OF MEDICINE (NLM), BETHESDA, MD, US. ABSTRACT NO. 77176612 D.M. ZUBAIROV ET AL. 'Blood coagulating properties of immobilized proteases'
- SOVIET PATENTS ABSTRACTS Section Ch, Week K49, 25. Januar 1984 Derwent Publications Ltd., London, GB; Class B04, AN 83-837652/49
- AMERICAN JOURNAL OF PHYSIOLOGY Bd. 201, Nr. 2, August 1961, US Seiten 298 - 302 R.H. LANDABURU ET AL. 'Prothrombin activation with trypsin as enzyme'
- DATABASE WPI Section Ch, Week 7943, Derwent Publications Ltd., London, GB; Class A96, AN 79-77442B (43)
- BIOCHIMICA ET BIOPHYSICA ACTA Bd. 329, Nr. 2, 5. Dezember 1973, AMSTERDAM, NL Seiten 221 - 232 W. KISIEL ET AL. 'The action of factor Xa, thrombin and trypsin on human factor II'

## Beschreibung

Die Erfindung betrifft ein Verfahren zur Aktivierung von Blutgerinnungsfaktoren mit Trypsin, sowie die Verwendung des Verfahrens zur Herstellung von Thrombin.

Die Blutgerinnung durchläuft eine Serie von aufeinanderfolgenden Reaktionen, in denen Blutgerinnungsfaktoren aktiviert werden, die wiederum subsequente Reaktionen katalysieren. Letztlich wird Fibrin durch die Wirkung von aktiviertem Prothrombin (Thrombin) aus Fibrinogen gebildet. Die Aktivierung der Blutgerinnungsfaktoren erfolgt meist durch die Proteolyse eines Zymogens zu einem proteolytisch wirksamen Enzym. Der Mechanismus dieser Aktivierungsreaktion ist zum Teil noch unbekannt, weshalb eine in vitro-Aktivierung von Blutgerinnungsfaktoren schwer kontrollierbar ist. Die Umwandlung von Prothrombin in Thrombin verläuft beispielsweise mit Faktor Xa und Calcium allein sehr langsam. Sie verläuft erst dann optimal, wenn wiederum ein Komplex mehrerer Faktoren (Prothombinase-Komplex) vorliegt. Neben Faktor Xa gehören zu diesem Komplex Faktor V, Phospholipide und Calcium. Der Faktor Xa spaltet das Prothrombinmolekül (Molekulargewicht 68.000 D) proteolytisch und generiert somit das aktive Enzym Thrombin (Molekulargewicht 30.000 D).

Die Plasmaprotease Thrombin ist ein multifunktionelles Enzym, welches nicht nur durch die Spaltung von Fibrinogen zu Fibrin koagulierend wirkt, sondern auch u.a. die Gerinnungsfaktoren V, VIII und XIII aktiviert und sein eigenes Proenzym (Prothrombin) spaltet. In der Therapie wird Thrombin alleine oder gemeinsam mit Fibrinogen zur Stillung von Blutungen oder in der Chirurgie zur Gewebeklebung eingesetzt.

Die Aktivierung von Prothrombin via dem Prothrombinasekomplex ist ex situ schwer nachzuahmen, weshalb es nicht an Versuchen fehlt, Thrombin durch die Einwirkung von Proteasen menschlichen oder tierischen Ursprungs zu generieren.

Untersuchungen zur Aktivierung von menschlichem Prothrombin durch bovinen Faktor Xa oder bovines Trypsin zeigten, daß die Aktivierung durch Trypsin via dieselben physiologischen Zwischenprodukte abläuft wie durch den Faktor Xa. Die Ausbeute liegt jedoch bei maximal 50 %, da im Fall von Trypsin die Proteolyse weitergeht, was zum Abbau von Thrombin bzw. zur Entstehung von low-molecular-weight-Produkten führt (Kisiel und Hanahan (1973), Biochim. Biophys. Acta 329, 221-232).

Die Ausbeute an Thrombin kann durch Zusätze wie Serumalbumin oder hohe Konzentrationen von Glycerin (50 %) verbessert werden (Landaburu et al. (1961), Am. J. Physiol. 201, 298-302). Allerdings muß das Produkt im Anschluß an die Aktivierungsreaktion in aufwendiger Weise von Trypsin und den diversen Zusätzen gereinigt werden, bevor es zu einer biologisch verträglichen Präparation verarbeitet werden kann.

Die Entfernung des Trypsins aus dem Reaktionsmedium könnte durch die Verwendung von immobilisiertem Enzym wesentlich vereinfacht werden. Die Verwendung von immobilisiertem Trypsin zur Aktivierung von Prothrombin galt jedoch bislang als unmöglich. Versuche zeigten, daß im Gegensatz zu löslichem Trypsin das immobilisierte Trypsin für die Aktivierung von Prothrombin zu Thrombin in Citratplasma nicht geeignet ist (Zubairov und Zinkevich (1976), Vosprosy meditsinskoi khimii 22(2), 187-91).

Der vorliegenden Erfindung liegt die Aufgabe zugrunde, ein einfaches Verfahren zur Aktivierung von Blutgerinnungsfaktoren zur Verfügung zu stellen, welches eine hohe Ausbeute an aktiviertem Enzym gewährleistet, ohne aufwendige Reinigungsschritte nach der Aktivierungsreaktion durchführen zu müssen.

Diese Aufgabe wird erfindungsgemäß durch ein Verfahren zur Aktivierung der Blutgerinnungsfaktoren Prothrombin, Faktor IX oder Faktor X mit Trypsin gelöst, welches darin besteht, daß der Blutgerinnungsfaktor aus einer Prothrombin-hältigen Fraktion gewonnen wird und mit an einem wasserunlöslichen Träger immobilisiertem Trypsin behandelt wird und das immobilisierte Trypsin nach der Aktivierung des Blutgerinnungsfaktors abgetrennt wird. Als wasserunlösliche Träger werden etwa Cellulose, Dextrane, Agarose, Acrylate oder Silikate verwendet.

Das Verfahren eignet sich besonders zur Aktivierung des Blutgerinnungsfaktors Prothrombin.

Eine vorteilhafte Ausführungsform der Erfindung besteht in der Durchführung der Aktivierung von Prothrombin, Faktor IX oder Faktor X bei einem pH-Wert von 5,8 bis 7,9, einer Leitfähigkeit von 5 bis 25 mS und einer Temperatur von 2 bis 45°C.

Das erfindungsgemäße Verfahren eignet sich besonders gut zur Gewinnung von Thrombin aus Prothrombin. Die kontrollierte Behandlung einer Fraktion, enthaltend gereinigtes Prothrombin, mit immobilisiertem Trypsin umfaßt die rasche und vollständige Abtrennung des Trypsins nach einer vorbestimmten Behandlungsdauer. Das Produkt enthält somit im wesentlichen keine Spaltprodukte. Auf den Zusatz üblicher Stabilisatoren, wie 50 % Glycerin, kann also verzichtet werden. Die Thrombin-hältige Fraktion kann in üblicher Weise zu einer pharmazeutischen Präparation verarbeitet werden.

Zur Herstellung eines aktivierten Enzyms aus einem Blutgerinnungsfaktor kann entweder der gereinigte Blutgerinnungsfaktor oder ein Blutgerinnungsfaktor-Komplex aktiviert werden, wobei das aktivierte Enzym im letzteren Fall vorteilhafterweise weitergereinigt wird. Thrombin kann also durch Aktivierung von gereinigtem Prothrombin oder von Prothrombinkomplex gewonnen werden.

Ein zusätzlicher Vorteil der Erfindung liegt darin, daß gleichzeitig mit der Aktivierung eines Blutgerinnungsfaktors eine Virusaktivität deutlich herabgesetzt wird, falls ein Ausgangsprodukt verwendet wird, welches aus einem mit Virus kontaminierten Pool stammt.

Die virusinaktivierende Wirkung des immobilisierten Trypsins in einer Blutgerinnungsfaktor-hältigen Fraktion war überraschend. Eine Behandlung mit immobilisierten neutralen Hydrolasen zur Inaktivierung von vermehrungsfähigen filtrierbaren Krankheitserregern war lediglich in Immunglobulin-G-hältigen Fraktionen bekannt (EP-A - 0 247 998).

Natürlich können im Rahmen des erfindungsgemäßen Verfahrens auch zusätzliche Maßnahmen zur Inaktivierung eventuell vorhandener infektiöser Agentien vorgenommen werden. So kann beispielsweise am befeuchteten lyophilisierten Ausgangsmaterial eine Dampf-Hitze-Behandlung vorgenommen werden.

Mit den nachfolgenden Beispielen wird die Erfindung noch näher erläutert.
1. Immobilisierung des Trypsins
   1,2 g Trypsin Type III (Sigma, Artikel Nr. T 8253) wurden an 350 ml (100 g Pulver) CNBr-aktivierte Sepharose 4B gemäß den Angaben der Hersteller (Fa. Pharmacia) gebunden. Mehr als 90 % des Trypsins wurden immobilisiert.
2. Aktivierung von Prothrombin in einer Prothrombinkomplex-hältigen Fraktion
   Aus 50 l menschlichem kryopräzipitatarmen Blutplasma wurde der Prothrombinkomplex nach dem bekannten Verfahren von Brummelhuis in "Methods of Plasma Fractionation" (J.M. Curling ed., Acad. Press 1980) durch Adsorption an einen Anionenaustauscher isoliert. Die Salzkonzentration der Prothrombin-enthaltenden Fraktion wurde durch Diafiltration auf 150 mM NaCl verringert und das Produkt gefriergetrocknet.
   Um eventuell enthaltene Krankheitserreger zu inaktivieren, wurde diese Fraktion nach AT-B - 385.657 in Anwesenheit von Wasserdampf 10 h bei 60°C und 1 h bei 80°C erhitzt.
   Das erhitzte Bulkpulver wurde zu 25 E Prothrombin/ml gelöst (Proteinkonzentration 12 mg/ml) und mit 1 ml Trypsin, immobilisiert an Sepharose, 150 min bei 25°C unter langsamem Rühren behandelt. Die Thrombin-Aktivität betrug 1980 E pro ml, die spezifische Aktivität 165 E/mg Protein.
3. Weiterreinigung des Thrombins aus 2.
   Das Thrombin wurde nach 2. hergestellt und über eine 45 ml S-Sepharose-Säule (Pharmacia) folgendermaßen weitergereinigt. Die Säule wurde mit 25 mM Na₃Citrat pH 6,2 äquilibriert. Thrombin (105.000 E in 25 mM Na₃Citrat) wurde an der S-Sepharose gebunden, mit dem gleichen Puffer gewaschen und mit 150 ml einer 450 mM NaCl-Lösung eluiert.
   Die Ausbeute an Thrombin war über 94 %, die spezifische Aktivität betrug 1735 E/mg Protein. Dieses Thrombin ließ sich in Anwesenheit von 9 g/l NaCl und 5 g/l Glycin zu einem stabilen Präparat gefriertrocknen.
4. Aktivierung von gereinigtem Prothrombin
   Die Gewinnung des dampfbehandelten Prothrombinkomplexes aus dem Plasma wurde nach 2. durchgeführt.
   Neben Prothrombin enthielt das virusinaktivierte Produkt noch Faktor IX und Faktor X. Durch eine chromatographische Trennung über sulfatisiertes Sephadex G50 (Pharmacia) (Miletich et al., (1980), Analyt. Biochem. 105, 304-310) wurden die Faktoren IX und X von Prothrombin abgetrennt. Nach Aktivierung des gereinigten Prothrombin zu Thrombin gemäß 2. war die spezifische Aktivität 745 E/mg.
5. Weiterreinigung des Thrombins aus 4.
   110.000 E Thrombin wurden nach 4. hergestellt und über eine 45 ml S-Sepharose-Säule weiter gereinigt. Nach der Reinigung hatte das Thrombin eine spezifische Aktivität von 3240 E/mg Protein.

## Patentansprüche

1. Verfahren zur Aktivierung eines der Blutgerinnungsfaktoren Prothrombin, Faktor IX oder Faktor X mit Trypsin, dadurch gekennzeichnet, daß der Blutgerinnungsfaktor aus einer Prothrombin-hältigen Fraktion gewonnen, mit an einem wasserunlöslichen Träger immobilisiertem Trypsin behandelt wird und das immobilisierte Trypsin nach der Aktivierung des Blutgerinnungsfaktors abgetrennt wird.

2. Verfahren nach Anspruch 1, dadurch gekennzeichnet, daß als Blutgerinnungsfaktor Prothrombin eingesetzt wird.

3. Verfahren nach Anspruch 1 oder 2, dadurch gekennzeichnet, daß die Aktivierung des Prothrombins, Faktors IX oder Faktors X bei einem pH-Wert von 5,8 bis 7,9, einer Leitfähigkeit von 5 bis 25 mS und einer Temperatur von 2 bis 45°C durchgeführt wird.

4. Verwendung des Verfahrens nach Anspruch 3 zur Gewinnung von Thrombin aus Prothrombin.

## Claims

1. A method of activating one of the blood coagulation factors prothrombin, factor IX or factor X by means of trypsin, characterized in that the blood coagulation factor is recovered from a prothrombin-containing fraction, is treated with trypsin immobilized on a water-insoluble carrier, and the immobilized trypsin is separated after activation of the blood coagulation factor.

2. A method according to claim 1, characterized in that prothrombin is used as blood coagulation factor.

3. A method according to claim 1 or 2, characterized in that the activation of prothrombin, factor IX or factor X is carried out at a pH of from 5.8 to 7.9, at a conductivity of from 5 to 25 mS and at a temperature of from 2 to 45°C.

4. The use of the method according to claim 3 for recovering thrombin from prothrombin.

## Revendications

1. Procédé pour l'activation de l'un des facteurs de coagulation du sang prothrombine, facteur IX ou facteur X avec la trypsine caractérisé en ce que le facteur de coagulation du sang est obtenu à partir d'une fraction contenant de la prothrombine, traité avec de la trypsine immobilisée sur un support insoluble dans l'eau et la trypsine immobilisée est séparée après l'activation du facteur de coagulation du sang.

2. Procédé selon la revendication 1 caractérisé en ce que la prothrombine est utilisée comme facteur de coagulation du sang.

3. Procédé selon la revendication 1 ou 2 caractérisé en ce que l'activation de la prothrombine, du facteur IX ou du facteur X est réalisée à un pH de 5,8 à 7,9, à une conductivité de 5 à 25 mS et à une température de 2 à 45°C.

4. Utilisation du procédé selon la revendication 3 pour l'obtention de thrombine à partir de prothrombine.
